(19) **Europäisches Patentamt / European Patent Office / Office européen des brevets**

(11) **EP 4 393 582 A1**

(12) # EUROPEAN PATENT APPLICATION
published in accordance with Art. 153(4) EPC

(43) Date of publication:
**03.07.2024 Bulletin 2024/27**

(21) Application number: **22859986.6**

(22) Date of filing: **06.06.2022**

(51) International Patent Classification (IPC):
**B01J 19/10** *(2006.01)*    **G01N 29/14** *(2006.01)*

(86) International application number:
**PCT/CN2022/097141**

(87) International publication number:
**WO 2023/024639 (02.03.2023 Gazette 2023/09)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **23.08.2021  CN 202110970195**

(71) Applicant: **Vinno Technology (Suzhou) Co., Ltd.
Jiangsu 215123 (CN)**

(72) Inventors:
• **GUO, Wei**
  **Suzhou, Jiangsu 215123 (CN)**
• **WU, Fanggang**
  **Suzhou, Jiangsu 215123 (CN)**

(74) Representative: **Winter, Brandl - Partnerschaft mbB
Alois-Steinecker-Straße 22
85354 Freising (DE)**

(54) **ULTRASONIC CAVITATION PARAMETER ADJUSTMENT METHOD AND ULTRASONIC CAVITATION APPARATUS**

(57) The present invention provides an ultrasonic cavitation parameter adjustment method and an ultrasonic cavitation device, wherein the parameter adjustment method comprises: receiving a selection signal of a detection frame, analyzing a position of the detection frame to determine a marked position; retrieving cavitation intensity data for the marked position from a preset collection of cavitation intensity distribution maps, selecting a cavitation intensity distribution map with maximum cavitation intensity data to obtain an optimal intensity distribution map; extracting cavitation output parameters corresponding to the optimal intensity distribution map, and outputting optimal ultrasonic emission parameters. The ultrasonic cavitation parameter adjustment method provided by the present invention allows the most appropriate cavitation output parameters to be retrieved regardless of the position of the selected detection frame, without the need for manual adjustments by the operator. It achieves the technical effects of improved efficiency, global automation, and ensuring optimal cavitation effects.

receiving a selection signal of a detection frame, analyzing a position of the detection frame to determine a marked position — *31*

retrieving cavitation intensity data for the marked position from a preset collection of cavitation intensity distribution maps, selecting a cavitation intensity distribution map with maximum cavitation intensity to obtain an optimal intensity distribution map — *32*

extracting cavitation output parameters corresponding to the optimal intensity distribution map and outputting optimal ultrasonic emission parameters — *33*

**FIG. 2**

EP 4 393 582 A1

**Description**

**CROSS-REFERENCE TO RELATED APPLICATION**

The application claims priority from Chinese Patent Application No. 202110970195.2, filed August 23, 2021, entitled "Ultrasonic Cavitation Parameter Adjustment Method and Ultrasonic Cavitation Device", all of which are incorporated herein by reference in their entirety.

**TECHNICAL FIELD**

The present invention relates to the field of ultrasonic detection and imaging technology, and more particularly to an ultrasonic cavitation parameter adjustment method and an ultrasonic cavitation device.

**BACKGROUND**

Ultrasonic cavitation technology is extensively applied in fields such as medicine and chemistry. It involves the application of ultrasonic wave energy to make tiny bubbles in a liquid vibrate and grow until they reach a certain energy threshold, after which the bubbles undergo rapid collapse and closure. This leads to the emulsification of liquids, liquefaction of gels, dispersion of solids, and the enhancement of drug absorption capabilities. In the process of adjusting ultrasonic cavitation parameters, prior art relies on manually adjusting the parameters based on the strongest Mechanical Index (MI) value in the current acoustic field. However, the solutions above only serve as a reference for controlling stable and transient cavitation. Given the numerous parameters influencing cavitation and the uneven distribution of ultrasonic energy, operators often cannot manually adjust the cavitation parameters to an optimal state.

**SUMMARY**

An object of the present invention is to provide an ultrasonic cavitation parameter adjustment method to solve technical problems in prior art that the adjustment of cavitation parameters is ineffective and cannot automatically adapt to the optimal parameters based on operator needs.

An object of the present invention is to provide an ultrasonic cavitation device.

In order to achieve one of the above-mentioned objects of the present invention, an embodiment of the present invention provides an ultrasonic cavitation parameter adjustment method, which comprises: receiving a selection signal of a detection frame, analyzing a position of the detection frame to determine a marked position; retrieving cavitation intensity data for the marked position from a preset collection of cavitation intensity distribution maps, selecting a cavitation intensity distribution map with maximum cavitation intensity data to obtain an optimal intensity distribution map; extracting cavitation output parameters corresponding to the optimal intensity distribution map, and outputting optimal ultrasonic emission parameters.

In an embodiment of the present invention, the ultrasonic cavitation parameter adjustment method further comprises: outputting at least two sets of cavitation trigger signals based on at least two sets of preset cavitation output parameters; analyzing and obtaining at least two sets of radio frequency data corresponding to the at least two sets of cavitation trigger signals, obtaining at least two cavitation intensity distribution maps based on the radio frequency data, and establishing a collection of cavitation intensity distribution maps.

In an embodiment of the present invention, the ultrasonic cavitation parameter adjustment method specifically comprises: calculating corresponding cavitation intensity data based on radio frequency data; mapping the cavitation intensity data to obtain pseudo-color data, and generating a cavitation intensity distribution map based on the pseudo-color data.

In an embodiment of the present invention, the ultrasonic cavitation parameter adjustment method specifically comprises: receiving echo data corresponding to a cavitation trigger signal; processing the echo data to obtain corresponding radio frequency data; extracting a subharmonic signal from the radio frequency data, calculating total pressure data at least at a first test point within a two-dimensional scanning plane; calculating cavitation intensity data for the first test point based on the total pressure data of the first test point and liquid pressure data of the first test point.

In an embodiment of the present invention, cavitation intensity data is the difference between total pressure data and liquid pressure data.

In an embodiment of the present invention, the ultrasonic cavitation parameter adjustment method specifically comprises: traversing cavitation intensity data to obtain maximum intensity data and minimum intensity data; calculating corresponding grayscale data based on the maximum intensity data, the minimum intensity data, and the cavitation intensity data; mapping the grayscale data to corresponding pseudo-color data based on an RGB mapping curve, and generating a cavitation intensity distribution map based on the pseudo-color data; wherein, the grayscale data satisfies:

$$g = \frac{255 \bullet (x - x_{\min})}{x_{\max} - x_{\min}}$$

, where $x$ represents the cavitation intensity data, $g$ represents the pseudo-color data corresponding to the cavitation intensity data, $x_{\max}$ represents the maximum intensity data, and $x_{\min}$ represents the minimum intensity data.

In an embodiment of the present invention, the ultrasonic cavitation parameter adjustment method specifically comprises: retrieving and calculating an average grayscale data at a marked position in each cavitation intensity distribution map within a collection of cavitation intensity distribution maps; selecting a cavitation intensity distribution map with maximum average grayscale data to obtain an optimal intensity distribution map; wherein, the average grayscale data is used to represent cavitation intensity at the marked position.

In an embodiment of the present invention, the ultrasonic cavitation parameter adjustment method further comprises: receiving an ultrasonic detection image; and superimposing an optimal intensity distribution map semi-transparently on the ultrasonic detection image to generate and output a cavitation display image.

In an embodiment of the present invention, the ultrasonic cavitation parameter adjustment method specifically comprises: selecting a region of interest in an ultrasonic detection image; setting a first weight for an optimal intensity distribution map, setting a second weight for the region of interest, and performing weighted blending of the optimal intensity distribution map and the region of interest to generate and output a cavitation display image; wherein the range of the first weight is 0 to 1, the range of the second weight is 0 to 1, and the sum of the first weight and the second weight equals 1.

In order to achieve one of the above-mentioned objects of the present invention, an embodiment of the present invention provides an ultrasonic cavitation device, which comprises: a main control module, an ultrasonic probe, an image processing module, a storage module, and a parameter setting module, wherein the ultrasonic cavitation device performs an ultrasonic cavitation parameter adjustment method according to any one of the above-mentioned technical solutions for parameter adjustment.

Compared to prior art, the ultrasonic cavitation parameter adjustment method provided by the present invention selects the cavitation intensity distribution map with the highest cavitation intensity data from the preset collection of cavitation intensity distribution maps by analyzing the cavitation intensity data at the marked position, and the ultrasonic emission parameters are adjusted to optimal ultrasonic emission parameters corresponding to the cavitation intensity distribution map with the highest cavitation intensity data. This approach ensures that the most appropriate cavitation output parameters for presetting are retrieved regardless of the position of the selected detection frame, without the need for manual adjustments by the operator. It achieves the technical effects of improved efficiency, global automation, and ensuring optimal cavitation effects.

## BRIEF DESCRIPTION OF THE DRAWINGS

FIG.1 shows a structural schematic diagram of an ultrasonic cavitation device in one embodiment of the invention.

FIG.2 shows a step schematic diagram of an ultrasonic cavitation parameter adjustment method in one embodiment of the invention.

FIG.3 shows a step schematic diagram of an ultrasonic cavitation parameter adjustment method in another embodiment of the invention.

FIG.4 shows a step schematic diagram of a preset process for the cavitation intensity distribution map of the ultrasonic cavitation parameter adjustment method in a first implement example of another embodiment of the invention.

FIG.5 shows a step schematic diagram for step 221 of the ultrasonic cavitation parameter adjustment method in a second implement example of another embodiment of the invention.

FIG.6 shows a step schematic diagram for step 222 of the ultrasonic cavitation parameter adjustment method in a third implement example of another embodiment of the invention.

FIG.7 shows a step schematic diagram of an ultrasonic cavitation parameter adjustment method in yet another embodiment of the invention.

FIG.8 shows a step schematic diagram of an ultrasonic cavitation parameter adjustment method in a first implement example of still another embodiment of the invention.

FIG.9 shows a step schematic diagram of an ultrasonic cavitation parameter adjustment method in a second implement example of still another embodiment of the invention.

## DETAILED DESCRIPTION

The present invention can be described in detail below with reference to the accompanying drawings and preferred embodiments. However, the embodiments are not intended to limit the invention, and the structural, method, or functional changes made by those skilled in the art in accordance with the embodiments are included in the scope of the present invention.

It should be noted that the term "comprising" and its variations are intended to cover non-exclusive inclusions, so a process, method, item, or device that includes a series of elements not only includes those elements but also includes other elements not explicitly listed, or elements inherent to such a process, method, item, or device. Furthermore, terms such as "first," "second," "third," etc., are used only for descriptive purposes and should not be interpreted as indicating or implying relative importance.

Ultrasonic cavitation is applied in medical and chemical fields, utilizing ultrasonic energy to cause cavitation nuclei to vibrate, grow, and accumulate energy under the influence of an acoustic field, leading to rapid collapse and closure. It enhances drug absorption capabilities at targeted lesion sites for therapeutic assistance or accelerates mixing between different media for faster physical reactions or chemical reactions. In one embodiment, cavitation nuclei refer to micron-sized or smaller bubbles within a biological body, specifically referring to tiny bubbles in liquids in one embodiment of the invention.

An ultrasonic cavitation device applies ultrasonic wave energy through an ultrasonic probe. Considering various factors such as probe volume and conditions of target environment, the sound field formed by the emission of ultrasonic waves from the ultrasonic probe is often uneven in intensity. This non-uniformity in the sound field, within the scanning range of the ultrasonic probe, results in varying Mechanical Index (MI) values in different regions. Consequently, the cavitation effects generated are also different. Thus, consistently establishing the sound field with the same parameters would lead to an inability to concentrate the focal point of the sound field on the target to be treated. This significantly weakens the auxiliary effect on therapy, physical reactions, or chemical reactions.

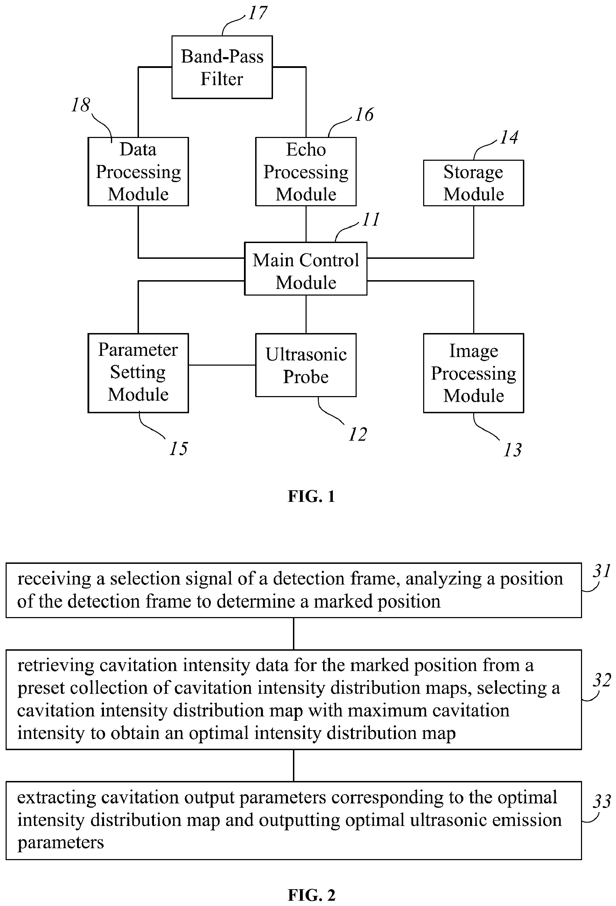

An ultrasonic cavitation device, as proposed in an embodiment of the present invention to solve technical problems in prior art, enhancing the effectiveness of ultrasonic cavitation. As shown in FIG.1, an ultrasonic cavitation device comprises a main control module 11, an ultrasonic probe 12, an image processing module 13, a storage module 14, and a parameter setting module 15. The device is configured to perform parameter adjustments through an ultrasonic cavitation parameter adjustment method to enhance the effectiveness of cavitation.

Specifically, the main control module 11 is coupled with a display device and an operating device in an external configuration to the main control module 11, receiving a detection frame selected by an operator on the display device via the operating device. The main control module 11 retrieves images meeting the criteria from the storage module 14 based on a marked position analyzed by the image processing module 13, processes these images in the image processing module 13, and extracts and outputs a corresponding parameter to the parameter setting module 15 for parameter setting. In some specific embodiments, the main control module 11 can be further configured to perform additional steps such as outputting cavitation trigger signals, receiving echo data, receiving radio frequency data, forwarding pseudo-color data, and receiving and outputting a cavitation display image. The additional steps can be adjusted according to the needs of those skilled in the art, and the present invention is not limited herein.

It is worth noting that, in one embodiment, the display device may specifically refer to the display screen of an upper computer or other computer, and the operating device may specifically refer to the computer's mainframe, keyboard and mouse set, or other devices. The display device is at least used for displaying images received and output by the ultrasonic cavitation device in the present invention, while the operating device is at least used for inputting signals indicating the selection of a detection fame.

The ultrasonic probe 12, coupled to the main control module 11 and the parameter setting module 15, is configured in one embodiment to integrate functions of cavitation output, cavitation feedback, and ultrasonic imaging. Alternatively, these functions can be assigned to separate devices in other embodiments. At least the part of the ultrasonic probe 12 responsible for cavitation output is coupled to the parameter setting module 15, while the part of the ultrasonic probe 12 responsible for cavitation feedback and ultrasonic imaging is coupled to the main control module 11. The ultrasonic probe 12 is further configured to accept cavitation output parameter settings from the parameter setting module 15, output cavitation effects, and receive data of cavitation feedback. In a specific embodiment, the ultrasonic probe 12 can also be configured to output and receive ultrasonic signals for imaging. The configuration of the ultrasonic probe 12 can be adjusted according to the needs of those skilled in the art.

The image processing module 13, coupled to the main control module 11, is configured to analyze and output the position of the detection frame within an image (or display), to analyze and output the cavitation output parameters corresponding to a selected cavitation intensity distribution map, and in one embodiment, also to calculate cavitation intensity distribution maps based on data of cavitation feedback, to establish and output a collection of cavitation intensity distribution maps, and to generate and output a cavitation display image. The image processing module 13 and the main control module 11 can be configured separately or integrated. In the case of separate configuration, the corresponding functions of one of the main control module 11 and the image processing module 13 can also be configured to be undertaken by the other of the main control module 11 and the image processing module 13.

The storage module 14, coupled to the main control module 11, is configured to receive retrieval commands from the main control module 11 for the data stored therein, to store the collection of cavitation intensity distribution maps, to store data related to the ultrasonic cavitation parameter adjustment method and to store a process image related to the ultrasonic cavitation parameter adjustment method. The parameter setting module 15, separately coupled to the main control module 11 and the ultrasonic probe 12, is configured to receive and output cavitation output parameters.

It should be understood that the explanation of modules above are differentiated by their respective functions and do not imply that the modules must be set separately in embodiments or that the functions the modules perform can only be undertaken by the corresponding module. Other embodiments arising from adjustments in modules and functions by those skilled in the art fall within the scope of the present invention.

In one embodiment, the ultrasonic cavitation device may further comprises sequentially coupled echo processing module 16, band-pass filter 17, and data processing module 18, with both the echo processing module 16 and the data processing module 18 separately coupled to the main control module 11. The echo processing module 16 is configured to process echo data forwarded by the main control module 11 to obtain radio frequency data. The band-pass filter 17 extracts a subharmonic signal from the radio frequency data and outputs. The data processing module 18 calculates cavitation intensity data based on the subharmonic signal and/or the radio frequency data, maps the cavitation intensity data to obtain pseudo-color data, and outputs. The modules mentioned above are not essential technical features for achieving the expected technical effects of the present invention and can be adjusted as needed. In the data and image mentioned above, some are data generated during the process, while others constitute the data intended as the final output. Before generating the final output data, there may also be other procedural data. Therefore, those skilled in the art can choose to use the procedural data as the output based on this.

To realize the technical effects of the present invention, one embodiment of the present invention provides an ultrasonic cavitation parameter adjustment method as shown in FIG.2, comprising:

> step 31: receiving a selection signal of a detection frame, analyzing a position of the detection frame to determine a marked position;
> step 32: retrieving cavitation intensity data for the marked position from a preset collection of cavitation intensity distribution maps, selecting a cavitation intensity distribution map with maximum cavitation intensity to obtain an optimal intensity distribution map;
> step 33: extracting cavitation output parameters corresponding to the optimal intensity distribution map and outputting optimal ultrasonic emission parameters.

The detection frame is an area selected by the operator on the display, which can be a frame with a certain area or a point or a set of points. The display is defined as an ultrasonic detection image in one embodiment, and the operator selects a region on the ultrasonic detection image as the detection frame, generating a corresponding selection signal. The process of analyzing the position of the detection frame can refer to the diagonal points of the detection frame or geometric center of the detection frame, establishing a Cartesian coordinate system on the display to represent the position in coordinate form, or setting a reference point on the display and determining the position of the detection frame based on the relative position between the detection frame and the reference point. Other embodiments resulting from modifications made by the skilled in art according to customary practices in the field fall within the scope of the present invention.

The marked position generated in the process above is stored, and a main control module 11 retrieves cavitation intensity data for each cavitation intensity distribution map in the collection of cavitation intensity distribution maps based on the marked position. If the marked position is a frame or a set of points with an area, the corresponding area on the cavitation intensity distribution map is scanned to obtain averaged cavitation intensity data for comparison or to obtain weighted cavitation intensity data for comparison. If the marked position is a single point, the corresponding position on the cavitation intensity distribution map is located at the single point, and the cavitation intensity data of the corresponding position is extracted for comparison.

It is noteworthy that in one embodiment, the cavitation intensity distribution map is defined as the intensity distribution conditions of cavitation effects produced by an ultrasonic probe under a specific combination of parameters (i.e., ultrasonic emission parameters for cavitation) setting on the display. Typically, cavitation intensity is stronger near the center of the ultrasonic probe and weaker away from the center of the ultrasonic probe. However, under different parameter settings, the intensity distribution conditions and area of regions with stronger and weaker cavitation intensity vary. In one embodiment, cavitation intensity distribution maps generated under different parameter settings are preset in a device for query and use. The collection of cavitation intensity distribution maps in one embodiment is defined as a set of multiple cavitation intensity distribution maps formed under different parameter settings, with the specific form adjustable according to the needs of the skilled in the art.

A distinction can be made between cavitation intensity data and cavitation output parameters. Cavitation intensity data represents the data status in different regions of the cavitation intensity distribution map, characterizing the cavitation intensity of different regions. Cavitation output parameters represent the parameter setting conditions for generating each cavitation intensity distribution map. Cavitation output parameters correspond to cavitation intensity distribution maps, and cavitation intensity data corresponds to a specific region within a cavitation intensity distribution map.

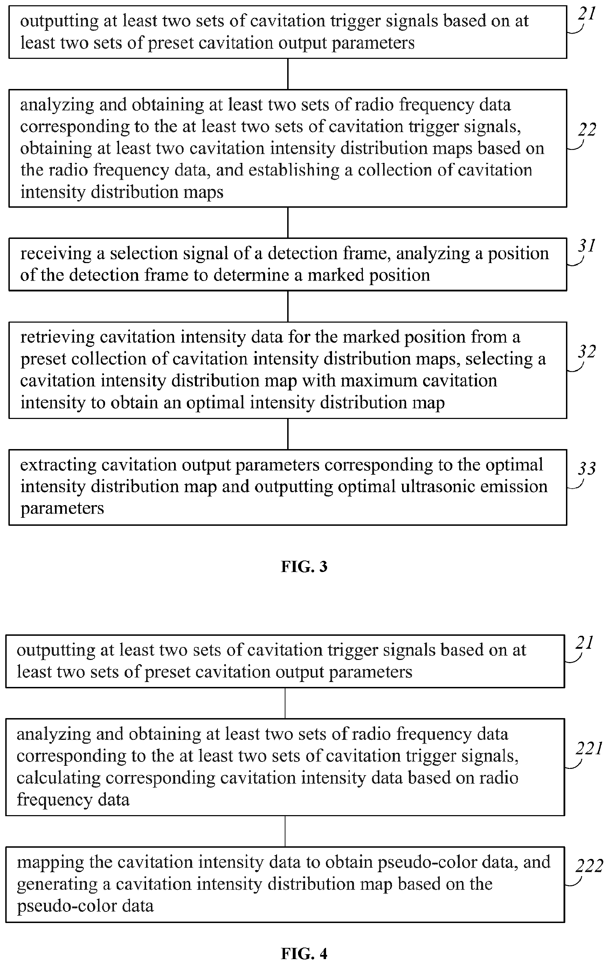

The aforementioned collection of cavitation intensity distribution maps can be obtained by pre-processing based on big data or by utilizing the ultrasonic cavitation device of the present invention for detection and calculation. For the latter case, another embodiment of the present invention provides an ultrasonic cavitation parameter adjustment method as shown in FIG.3, comprising:

> step 21: outputting at least two sets of cavitation trigger signals based on at least two sets of preset cavitation output parameters;
> step 22: analyzing and obtaining at least two sets of radio frequency data corresponding to the at least two sets of cavitation trigger signals, obtaining at least two cavitation intensity distribution maps based on the radio frequency data, and establishing a collection of cavitation intensity distribution maps;
step 31: receiving a selection signal of a detection frame, analyzing a position of the detection frame to determine a marked position;
step 32: retrieving cavitation intensity data for the marked position from a preset collection of cavitation intensity distribution maps, selecting a cavitation intensity distribution map with maximum cavitation intensity to obtain an optimal intensity distribution map;
step 33: extracting cavitation output parameters corresponding to the optimal intensity distribution map and outputting optimal ultrasonic emission parameters.

Cavitation trigger signals contain data from ultrasonic emission parameters that affect the effectiveness of cavitation, such as emission frequency, emission cycle, acoustic power, and PRF (Pulse Repetition Frequency), and other relevant information. Between the two sets of ultrasonic emission parameters and corresponding cavitation trigger signals, at least one of the aforementioned information differs, resulting in obtaining at least two cavitation intensity distribution maps with different intensity distribution conditions.

The feedback form of cavitation in one embodiment mainly involves radio frequency data, and the cavitation intensity distribution maps are generated by analyzing and calculating based on the radio frequency data corresponding to the cavitation trigger signals. If other feedback forms are implemented, the steps from step 21 to step 22 can be adjusted accordingly, as long as the steps are sufficient to produce two distinct cavitation intensity distribution maps. Moreover, the order of step 21, step 22 and steps from step 31 to step 33 can be adjusted as long as the collection of cavitation intensity distribution maps is preset before executing step 32, thereby achieving the intended technical effect of the present invention.

Preferably, to avoid unnecessary targets interfering with the feedback signals (such as echo signals) of cavitation effects, step 21 and step 22 can be executed in an environment distinct from step 31 to step 32. The aforementioned environment, in one embodiment, is defined as a pure water environment without air bubbles and injected with ultrasound contrast agents , which can be set up in a water tank. During operation, the detection surface (an ultrasonic transducer array in one embodiment) of the ultrasonic probe 12 is aligned parallel to and submerged in the water surface. Those skilled in the art can use other technical means to achieve the same effect as the above technical solution.

The aforementioned cavitation intensity distribution maps can be easily configured as grayscale images, representing the intensity distribution conditions of cavitation effects through the numerical magnitude of grayscale data. To enhance the display of the cavitation intensity distribution maps, a first implement example of the embodiment is further provided as shown in FIG.4, comprising:

step 21: outputting at least two sets of cavitation trigger signals based on at least two sets of preset cavitation output parameters;
step 221: analyzing and obtaining at least two sets of radio frequency data corresponding to the at least two sets of cavitation trigger signals, calculating corresponding cavitation intensity data based on radio frequency data;
step 222, mapping the cavitation intensity data to obtain pseudo-color data, and generating a cavitation intensity distribution map based on the pseudo-color data;
step 31: receiving a selection signal of a detection frame, analyzing a position of the detection frame to determine a marked position;
step 32: retrieving cavitation intensity data for the marked position from a preset collection of cavitation intensity distribution maps, selecting a cavitation intensity distribution map with maximum cavitation intensity to obtain an optimal intensity distribution map;
step 33: extracting cavitation output parameters corresponding to the optimal intensity distribution map and outputting optimal ultrasonic emission parameters.

The mapping process for pseudo-color data comprises: using cavitation intensity data as code values to look up corresponding R, G, B intensity values in a color look-up table (CLUT), thereby performing the mapping. Since the pseudo-color data contains cavitation intensity data and human eyes are more sensitive to colors than to grayscale, the resulting cavitation intensity distribution maps can more prominently represent the distribution conditions of cavitation intensity. The resulting cavitation intensity distribution maps or the collection of cavitation intensity distribution maps can be stored in the storage module 14 and can also be output to a display device for display.

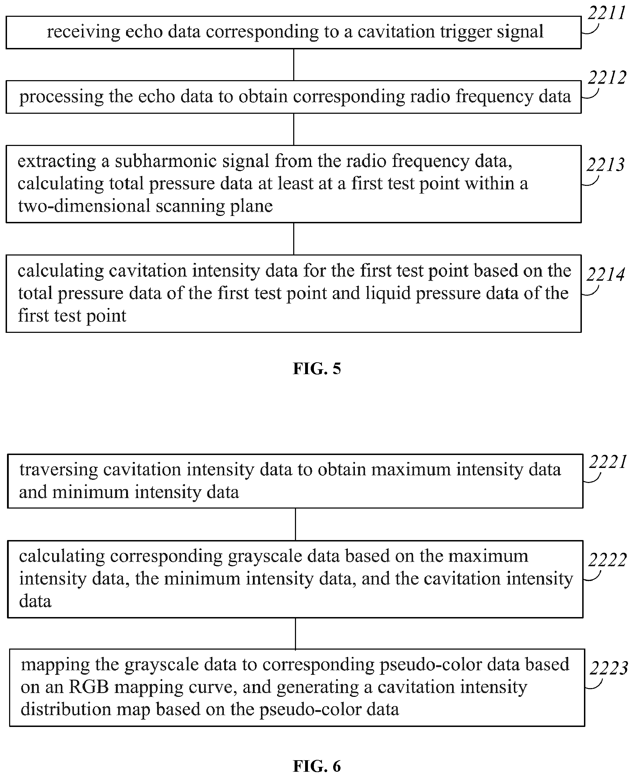

Cavitation trigger signals are output from the ultrasonic probe 12, which then performs cavitation effects and receives echo signals representing the effectiveness of the cavitation effects. The collection of echo signals is time-based, and the establishment of cavitation intensity distribution maps requires the relative positional relationship between the depth and lateral dimensions. Regarding how to convert data of echo signals (i.e., echo data) into data usable for generating cavitation intensity distribution maps, there can be multiple implementations. A second implement example of the embodiment is further provided as shown in FIG.5, comprising:

step 21: outputting at least two sets of cavitation trigger signals based on at least two sets of preset cavitation output parameters;
step 2211: receiving echo data corresponding to a cavitation trigger signal;
step 2212: processing the echo data to obtain corresponding radio frequency data;

step 2213: extracting a subharmonic signal from the radio frequency data, calculating total pressure data at least at a first test point within a two-dimensional scanning plane;

step 2214: calculating cavitation intensity data for the first test point based on the total pressure data of the first test point and liquid pressure data of the first test point;

step 222, mapping the cavitation intensity data to obtain pseudo-color data, and generating a cavitation intensity distribution map based on the pseudo-color data;

step 31: receiving a selection signal of a detection frame, analyzing a position of the detection frame to determine a marked position;

step 32: retrieving cavitation intensity data for the marked position from a preset collection of cavitation intensity distribution maps, selecting a cavitation intensity distribution map with maximum cavitation intensity to obtain an optimal intensity distribution map;

step 33: extracting cavitation output parameters corresponding to the optimal intensity distribution map and outputting optimal ultrasonic emission parameters.

The process of processing echo data into radio frequency data can have specific configurations. For example, in one embodiment, beamforming can be performed to the echo data, and the received beam formed as a result can be used as the radio frequency data. The subharmonic signal is extracted from the radio frequency data using a band-pass filter 17. In a static water environment, the amplitude changes of the subharmonic signal exhibits a strong correlation with the pressure within the water body, thus, in one embodiment, enabling the calculation of total pressure data at least at a first test point within the water body utilizing the amplitude variations of the subharmonic signal.

It should be noted that, in the embodiment, the subharmonic signal specifically refers to the subharmonic component distinguished from the first harmonic component or the second harmonic component, which exhibits a better correlation with pressure, leading to more accurate estimation of total pressure data.

The relationship between cavitation intensity data and total pressure data can have different embodiments. In one implement example, the cavitation intensity data is set as the difference between total pressure data and the liquid pressure data:

$$P_{final} = P_{ultrasound} + P_{bubble} = P_{cal} - P_{water} ,$$

where $P_{final}$ represents equivalent cavitation intensity data, $P_{ultrasound}$ represents acoustic pressure emitted by the ultrasonic probe 12, $P_{bubble}$ represents a pressure generated by the resonance of cavitation nuclei (e.g., tiny bubbles), i.e., actual cavitation intensity data, $P_{cal}$

represents total pressure data, and $P_{water}$ represents liquid pressure data. The rationale behind the aforementioned formula is that the actual cavitation intensity data $P_{bubble}$ has a strong correlation with parameters affecting acoustic pressure $P_{ultrasound}$, especially with Peak Rarefactional Pressure (PRP), Pulse Repetition Frequency (PRF), and Pulse Duration (PD). Therefore, it is considered that there is a corresponding relationship between actual cavitation intensity data $P_{bubble}$ and acoustic pressure $P_{ultrasound}$, and acoustic pressure $P_{ultrasound}$ can be converted into parameters containing actual cavitation intensity data $P_{bubble}$ . Alternatively, equivalent cavitation intensity data $P_{final}$ can be defined to represent the current cavitation intensity.

Further, at least the first test point during operation is simultaneously affected by liquid pressure (i.e., liquid pressure data $P_{water}$ ), actual cavitation intensity (i.e., actual cavitation intensity data $P_{bubble}$), and acoustic pressure $P_{ultrasound}$, i.e., affected by liquid pressure (i.e., liquid pressure data $P_{water}$) and equivalent cavitation intensity data $P_{final}$, constituting the total pressure (i.e., total pressure data $P_{cal}$ ) detected by the ultrasonic probe 12. Thus, through the above formula, cavitation intensity data representing cavitation intensity (i.e., the equivalent cavitation intensity data) can be simply calculated by subtraction operations.

It should be noted that the liquid pressure (i.e., liquid pressure data $P_{water}$) can be measured in several other ways. In one embodiment, step 2213 and step 2214 can be divided into two phases. In the first phase, the ultrasonic probe 12 does not apply cavitation effects to the water body, only measuring liquid pressure data $P_{water}$ at a first test point. In the second phase, the ultrasonic probe 12 applies cavitation effects to the water body, measuring the total pressure data $P_{cal}$ at the first test point. The equivalent cavitation intensity data representing the cavitation effects is then obtained by subtracting the data measured in the two phases(i.e., liquid pressure data $P_{water}$ and total pressure data $P_{cal}$).

The present invention discloses a technical solution for invoking cavitation intensity data for comparison, thereby determining the cavitation output parameters. In fact, the present invention provides technical insights into processing original cavitation intensity data (i.e., the equivalent cavitation intensity data) to achieve the objectives of the present invention. Further, a third implement example of the embodiment provides a technical solution for generating a cavitation intensity distribution map by mapping grayscale data to pseudo-color data, as shown in FIG.6, comprising:

step 21: outputting at least two sets of cavitation trigger signals based on at least two sets of preset cavitation output parameters;

step 221: analyzing and obtaining at least two sets of radio frequency data corresponding to the at least two sets of cavitation trigger signals, calculating corresponding cavitation intensity data based on radio frequency data;

step 2221: traversing cavitation intensity data to obtain maximum intensity data and minimum intensity data;

step 2222: calculating corresponding grayscale data based on the maximum intensity data, the minimum intensity data, and the cavitation intensity data;

step 2223: mapping the grayscale data to corresponding pseudo-color data based on an RGB mapping curve, and generating a cavitation intensity distribution map based on the pseudo-color data;

step 31: receiving a selection signal of a detection frame, analyzing a position of the detection frame to determine a marked position;

step 32: retrieving cavitation intensity data for the marked position from a preset collection of cavitation intensity distribution maps, selecting a cavitation intensity distribution map with maximum cavitation intensity to obtain an optimal intensity distribution map;

step 33: extracting cavitation output parameters corresponding to the optimal intensity distribution map and outputting optimal ultrasonic emission parameters.

The grayscale data satisfies:

$$g = \frac{255 \bullet (x - x_{min})}{x_{max} - x_{min}}$$

, where $x$ represents the cavitation intensity data, $g$ represents the pseudo-color data corresponding to the cavitation intensity data, $x_{max}$ represents the maximum intensity data, and $x_{min}$ represents the minimum intensity data. By processing in this manner, cavitation intensity data with a wide range can be initially mapped to grayscale data within the range of 0 to 255. Subsequently, pseudo-color data is generated by utilizing methods such as table lookup or curve mapping to map the grayscale data. Ultimately, a cavitation intensity distribution map is generated based on all pseudo-color data corresponding to the current cavitation output parameters.

It should be noted that since subsequent steps require looking up cavitation intensity data in the cavitation intensity distribution map, the mapping process for generating the cavitation intensity distribution maps does not lose the cavitation intensity data. Alternatively, a separate step can be set before step 32 to convert pseudo-color data or grayscale data back into cavitation intensity data, or step 32 can be modified to retrieve grayscale data or pseudo-color data instead of cavitation intensity data, to achieve the desired technical effect.

The cavitation intensity distribution map generated through the above steps are displayed in color, with areas of high cavitation intensity data value (which represents strong cavitation effects) shown in deep red or red, and areas of low cavitation intensity data value (which represents weak cavitation effects) shown in purple or blue-purple. Areas with cavitation effects ranging between strong cavitation and weak cavitation are displayed in colors such as yellow or green.

Alternatively, other implementations for mapping uniformly distributing data to form pseudo-color data and further generate pseudo-color images are also involved in the present invention. Those skilled in the art can alternatively implement the other implementations mentioned above in the technical solutions provided for the present invention as described earlier.

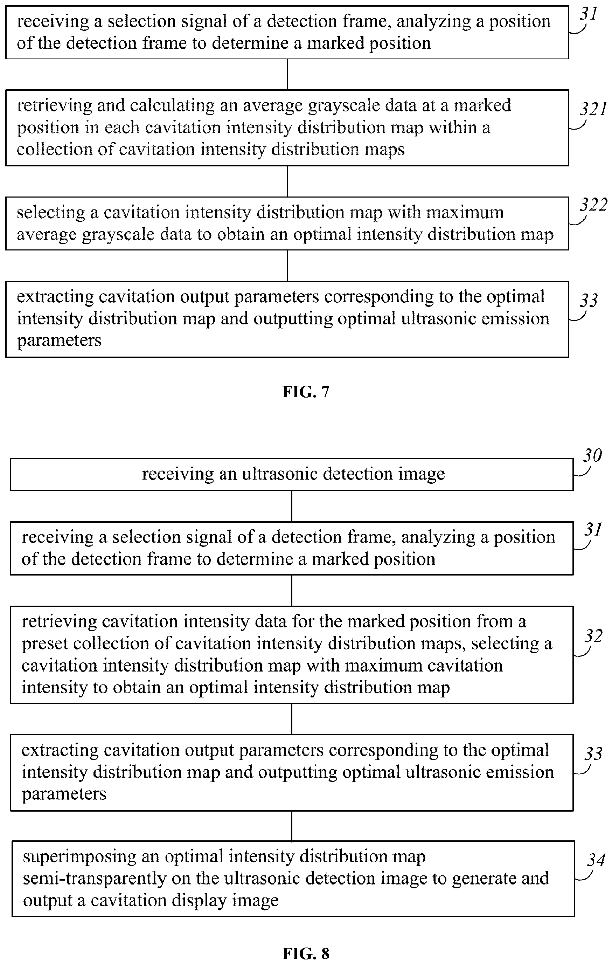

For the technical solution of using grayscale data instead of cavitation intensity data for retrieval and selection, yet another embodiment of the present invention provides an ultrasonic cavitation parameter adjustment method as shown in FIG.7, comprising:

step 21: outputting at least two sets of cavitation trigger signals based on at least two sets of preset cavitation output parameters;

step 221: analyzing and obtaining at least two sets of radio frequency data corresponding to the at least two sets of cavitation trigger signals, calculating corresponding cavitation intensity data based on radio frequency data;

step 2221: traversing cavitation intensity data to obtain maximum intensity data and minimum intensity data;

step 2222: calculating corresponding grayscale data based on the maximum intensity data, the minimum intensity data, and the cavitation intensity data;

step 2223: mapping the grayscale data to corresponding pseudo-color data based on an RGB mapping curve, and generating a cavitation intensity distribution map based on the pseudo-color data;

step 31: receiving a selection signal of a detection frame, analyzing a position of the detection frame to determine a marked position;

step 321: retrieving and calculating an average grayscale data at a marked position in each cavitation intensity distribution map within a collection of cavitation intensity distribution maps;

step 322: selecting a cavitation intensity distribution map with maximum average grayscale data to obtain an optimal intensity distribution map;

step 33: extracting cavitation output parameters corresponding to the optimal intensity distribution map and outputting optimal ultrasonic emission parameters.

The average grayscale data is used to represent cavitation intensity at the marked position.

It should be noted that calculating the average is just one approach provided in one embodiment of the present invention. In other embodiments, algorithms such as weighted fusion can be implemented to achieve desired technical effects. Additionally, the detailed step 321 and step 322 can be executed based on grayscale data calculated from cavitation intensity data, so the pseudo-color data mapping process in step 2223 is not a necessary technical feature of the embodiment.

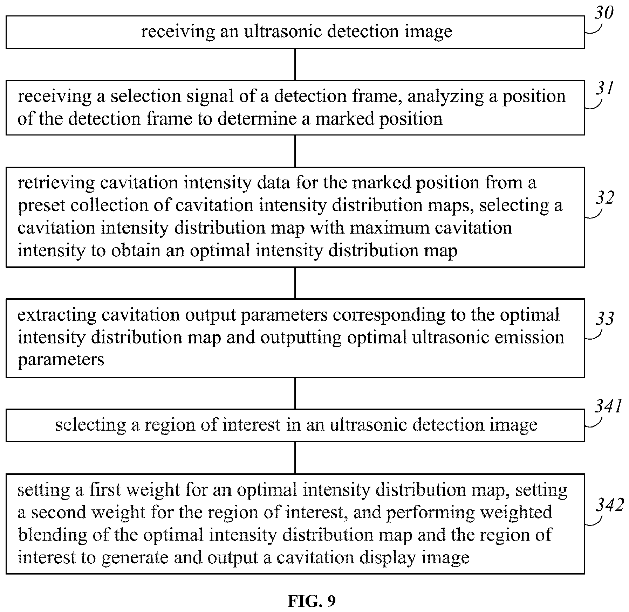

Under a specific working condition where the display is defined as an ultrasonic detection image, the present invention provides still another embodiment, which includes a first implement example as shown in FIG.8 and a second implement example as shown in FIG.9.

An ultrasonic cavitation parameter adjustment method provided by the first implement example of the still another embodiment specifically comprises:

> step 30: receiving an ultrasonic detection image;
> step 31: receiving a selection signal of a detection frame, analyzing a position of the detection frame to determine a marked position;
> step 32: retrieving cavitation intensity data for the marked position from a preset collection of cavitation intensity distribution maps, selecting a cavitation intensity distribution map with maximum cavitation intensity to obtain an optimal intensity distribution map;
> step 33: extracting cavitation output parameters corresponding to the optimal intensity distribution map and outputting optimal ultrasonic emission parameters;
> step 34: superimposing an optimal intensity distribution map semi-transparently on the ultrasonic detection image to generate and output a cavitation display image.

The ultrasonic detection image, especially a B-mode ultrasonic image, are usually black and white. Superimposing a semi-transparent and color-rendered optimal intensity distribution map on the ultrasonic detection image clearly displays the relationship between the current detection target and the distribution of cavitation intensity, helping the operator understand the current detection status (or imaging status) and the application of cavitation effects. Other methods of generating a cavitation display image that combines the features of the optimal intensity distribution map and the ultrasonic detection image can also achieve expected technical effects.

Furthermore, the order of step 30 and step 34 is not restricted. The desired technical effects can be achieved as long as step 30 is executed before step 34, and step 34 is executed after step 32.

An ultrasonic cavitation parameter adjustment method provided by the second implement example of the still another embodiment specifically comprises:

> step 30: receiving an ultrasonic detection image;
> step 31: receiving a selection signal of a detection frame, analyzing a position of the detection frame to determine a marked position;
> step 32: retrieving cavitation intensity data for the marked position from a preset collection of cavitation intensity distribution maps, selecting a cavitation intensity distribution map with maximum cavitation intensity to obtain an optimal intensity distribution map;
> step 33: extracting cavitation output parameters corresponding to the optimal intensity distribution map and outputting optimal ultrasonic emission parameters;
> step 341: selecting a region of interest in an ultrasonic detection image;
> step 342: setting a first weight for an optimal intensity distribution map, setting a second weight for the region of interest, and performing weighted blending of the optimal intensity distribution map and the region of interest to generate and output a cavitation display image.

The range of the first weight is 0 to 1, the range of the second weight is 0 to 1, and the sum of the first weight and the second weight equals 1. By adjusting the values of the first weight and the second weight, the transparency of the optimal intensity distribution map can be altered, thereby changing the display effect of the cavitation display image.

The region of interest (ROI) mentioned above is used in the fields of image processing and machine vision, defined as an area to be processed which is outlined from a processed image (in one embodiment, from an ultrasonic detection image) in the form of a rectangle, circle, ellipse, etc. It can be applied in software like Halcon, OpenCV, Matlab, etc. In a specific embodiment, the entire ultrasonic detection image can be selected as the region of interest, or the region determined by a detection frame or the region input by the operator can be selected as the region of interest. In another specific embodiment, the ultrasonic detection image, the cavitation intensity distribution map, and the cavitation display image have the same dimensions, preferably matching the size of the display.

Regarding the multiple embodiments and implement examples of the ultrasonic cavitation parameter adjustment method provided by the present invention, the order of steps can be adjusted according to the needs of the skilled in the art, without affecting the technical effects. It is important to note that the various ultrasonic cavitation parameter adjustment methods provided by the present invention should not be regarded as necessarily isolated from each other. The steps of each embodiment or implement example can certainly be combined and/or replaced to form new embodiments that fall within the scope of protection of the present invention.

In summary, the ultrasonic cavitation parameter adjustment method provided by the present invention selects a cavitation intensity distribution map with the highest cavitation intensity data from the preset collection of cavitation intensity distribution maps by analyzing the cavitation intensity data at the marked position, and the ultrasonic emission parameters are adjusted to optimal ultrasonic emission parameters corresponding to the cavitation intensity distribution map with the highest cavitation intensity data. This approach ensures that the most appropriate cavitation output parameters for presetting are retrieved regardless of the position of the selected detection frame, without the need for manual adjustments by the operator. It achieves the technical effects of improved efficiency, global automation, and ensuring optimal cavitation effects.

It should be understood that, although the description is described in terms of embodiments, not every embodiment merely comprises an independent technical solution. The description is described in this manner for clarity, and those skilled in the art should consider the description as a whole, and the technical solutions in each embodiment may also be combined as appropriate to form other embodiments that can be understood by those skilled in the art.

The series of detailed descriptions set forth above are only specific descriptions of feasible embodiments of the present invention and are not intended to limit the scope of protection of the present invention. Equivalent embodiments or modifications made within the spirit of the present invention should be included within the protection scope of the present invention.

## Claims

1. An ultrasonic cavitation parameter adjustment method, which comprises:

    receiving a selection signal of a detection frame, analyzing a position of the detection frame to determine a marked position;
    retrieving cavitation intensity data for the marked position from a preset collection of cavitation intensity distribution maps, selecting a cavitation intensity distribution map with maximum cavitation intensity data to obtain an optimal intensity distribution map; and
    extracting cavitation output parameters corresponding to the optimal intensity distribution map, and outputting optimal ultrasonic emission parameters.

2. The ultrasonic cavitation parameter adjustment method of claim 1, which further comprises:

    outputting at least two sets of cavitation trigger signals based on at least two sets of preset cavitation output parameters; and
    analyzing and obtaining at least two sets of radio frequency data corresponding to the at least two sets of cavitation trigger signals, obtaining at least two cavitation intensity distribution maps based on the radio frequency data, and establishing a collection of cavitation intensity distribution maps.

3. The ultrasonic cavitation parameter adjustment method of claim 2, which specifically comprises:

    calculating corresponding cavitation intensity data based on radio frequency data; and
    mapping the cavitation intensity data to obtain pseudo-color data, and generating a cavitation intensity distribution map based on the pseudo-color data.

4. The ultrasonic cavitation parameter adjustment method of claim 3, which specifically comprises:

    receiving echo data corresponding to a cavitation trigger signal;
    processing the echo data to obtain corresponding radio frequency data;
    extracting a subharmonic signal from the radio frequency data, calculating total pressure data at least at a first test point within a two-dimensional scanning plane; and
    calculating cavitation intensity data for the first test point based on the total pressure data of the first test point and liquid pressure data of the first test point.

5. The ultrasonic cavitation parameter adjustment method of claim 4, wherein cavitation intensity data is the difference between total pressure data and liquid pressure data.

6. The ultrasonic cavitation parameter adjustment method of claim 3, which specifically comprises:

    traversing cavitation intensity data to obtain maximum intensity data and minimum intensity data;
    calculating corresponding grayscale data based on the maximum intensity data, the minimum intensity data, and the cavitation intensity data; and
    mapping the grayscale data to corresponding pseudo-color data based on an RGB mapping curve, and generating a cavitation intensity distribution map based on the pseudo-color data; wherein, the grayscale data satisfies:

$$g = \frac{255 \bullet (x - x_{min})}{x_{max} - x_{min}}$$

, $x_{min}$, where $x$ represents the cavitation intensity data, $g$ represents the pseudo-color data corresponding to the cavitation intensity data, $x_{max}$ represents the maximum intensity data, and $x_{min}$ represents the minimum intensity data.

7. The ultrasonic cavitation parameter adjustment method of claim 6, which specifically comprises:

retrieving and calculating an average grayscale data at a marked position in each cavitation intensity distribution map within a collection of cavitation intensity distribution maps; and

selecting a cavitation intensity distribution map with maximum average grayscale data to obtain an optimal intensity distribution map; wherein, the average grayscale data is used to represent cavitation intensity at the marked position.

8. The ultrasonic cavitation parameter adjustment method of claim 1, which further comprises:

receiving an ultrasonic detection image; and superimposing an optimal intensity distribution map semi-transparently on the ultrasonic detection image to generate and output a cavitation display image.

9. The ultrasonic cavitation parameter adjustment method of claim 8, which specifically comprises:

selecting a region of interest in an ultrasonic detection image; and

setting a first weight for an optimal intensity distribution map, setting a second weight for the region of interest, and performing weighted blending of the optimal intensity distribution map and the region of interest to generate and output a cavitation display image; wherein the range of the first weight is 0 to 1, the range of the second weight is 0 to 1, and the sum of the first weight and the second weight equals 1.

10. An ultrasonic cavitation device, which comprises: a main control module, an ultrasonic probe, an image processing module, a storage module, and a parameter setting module, wherein the ultrasonic cavitation device performs an ultrasonic cavitation parameter adjustment method according to any one of claims 1-9 for parameter adjustment.

17

Band-Pass
Filter

18

Data
Processing
Module

16

Echo
Processing
Module

14

Storage
Module

11

Main Control
Module

Parameter
Setting
Module

Ultrasonic
Probe

Image
Processing
Module

15

12

13

**FIG. 1**

| receiving a selection signal of a detection frame, analyzing a position of the detection frame to determine a marked position | 31 |
|---|---|
| retrieving cavitation intensity data for the marked position from a preset collection of cavitation intensity distribution maps, selecting a cavitation intensity distribution map with maximum cavitation intensity to obtain an optimal intensity distribution map | 32 |
| extracting cavitation output parameters corresponding to the optimal intensity distribution map and outputting optimal ultrasonic emission parameters | 33 |

**FIG. 2**

outputting at least two sets of cavitation trigger signals based on at least two sets of preset cavitation output parameters ⌐21

analyzing and obtaining at least two sets of radio frequency data corresponding to the at least two sets of cavitation trigger signals, obtaining at least two cavitation intensity distribution maps based on the radio frequency data, and establishing a collection of cavitation intensity distribution maps ⌐22

receiving a selection signal of a detection frame, analyzing a position of the detection frame to determine a marked position ⌐31

retrieving cavitation intensity data for the marked position from a preset collection of cavitation intensity distribution maps, selecting a cavitation intensity distribution map with maximum cavitation intensity to obtain an optimal intensity distribution map ⌐32

extracting cavitation output parameters corresponding to the optimal intensity distribution map and outputting optimal ultrasonic emission parameters ⌐33

**FIG. 3**

outputting at least two sets of cavitation trigger signals based on at least two sets of preset cavitation output parameters ⌐21

analyzing and obtaining at least two sets of radio frequency data corresponding to the at least two sets of cavitation trigger signals, calculating corresponding cavitation intensity data based on radio frequency data ⌐221

mapping the cavitation intensity data to obtain pseudo-color data, and generating a cavitation intensity distribution map based on the pseudo-color data ⌐222

**FIG. 4**

receiving echo data corresponding to a cavitation trigger signal | 2211

processing the echo data to obtain corresponding radio frequency data | 2212

extracting a subharmonic signal from the radio frequency data, calculating total pressure data at least at a first test point within a two-dimensional scanning plane | 2213

calculating cavitation intensity data for the first test point based on the total pressure data of the first test point and liquid pressure data of the first test point | 2214

**FIG. 5**

traversing cavitation intensity data to obtain maximum intensity data and minimum intensity data | 2221

calculating corresponding grayscale data based on the maximum intensity data, the minimum intensity data, and the cavitation intensity data | 2222

mapping the grayscale data to corresponding pseudo-color data based on an RGB mapping curve, and generating a cavitation intensity distribution map based on the pseudo-color data | 2223

**FIG. 6**

| receiving a selection signal of a detection frame, analyzing a position of the detection frame to determine a marked position | 31 |
| --- | --- |

| retrieving and calculating an average grayscale data at a marked position in each cavitation intensity distribution map within a collection of cavitation intensity distribution maps | 321 |

| selecting a cavitation intensity distribution map with maximum average grayscale data to obtain an optimal intensity distribution map | 322 |

| extracting cavitation output parameters corresponding to the optimal intensity distribution map and outputting optimal ultrasonic emission parameters | 33 |

**FIG. 7**

| receiving an ultrasonic detection image | 30 |
| --- | --- |

| receiving a selection signal of a detection frame, analyzing a position of the detection frame to determine a marked position | 31 |

| retrieving cavitation intensity data for the marked position from a preset collection of cavitation intensity distribution maps, selecting a cavitation intensity distribution map with maximum cavitation intensity to obtain an optimal intensity distribution map | 32 |

| extracting cavitation output parameters corresponding to the optimal intensity distribution map and outputting optimal ultrasonic emission parameters | 33 |

| superimposing an optimal intensity distribution map semi-transparently on the ultrasonic detection image to generate and output a cavitation display image | 34 |

**FIG. 8**

15

| receiving an ultrasonic detection image | 30 |
| :-- | :-- |

| receiving a selection signal of a detection frame, analyzing a position of the detection frame to determine a marked position | 31 |

| retrieving cavitation intensity data for the marked position from a preset collection of cavitation intensity distribution maps, selecting a cavitation intensity distribution map with maximum cavitation intensity to obtain an optimal intensity distribution map | 32 |

| extracting cavitation output parameters corresponding to the optimal intensity distribution map and outputting optimal ultrasonic emission parameters | 33 |

| selecting a region of interest in an ultrasonic detection image | 341 |

| setting a first weight for an optimal intensity distribution map, setting a second weight for the region of interest, and performing weighted blending of the optimal intensity distribution map and the region of interest to generate and output a cavitation display image | 342 |

FIG. 9

## INTERNATIONAL SEARCH REPORT

| International application No. |
|---|
| **PCT/CN2022/097141** |

**A. CLASSIFICATION OF SUBJECT MATTER**

B01J 19/10(2006.01)i; G01N 29/14(2006.01)i

According to International Patent Classification (IPC) or to both national classification and IPC

**B. FIELDS SEARCHED**

Minimum documentation searched (classification system followed by classification symbols)

B01J, G01N

Documentation searched other than minimum documentation to the extent that such documents are included in the fields searched

Electronic data base consulted during the international search (name of data base and, where practicable, search terms used)

CNTXT; USTXT; CNABS; DWPI; VEN; CNKI: 飞依诺, 郭威, 吴方刚, 超声, 空化, 强度, 声强, 参数, 调整, 控制, ultrasonic, cavitation, intensity, strength, parameter, adjust+, control+, retriev+

**C. DOCUMENTS CONSIDERED TO BE RELEVANT**

| Category* | Citation of document, with indication, where appropriate, of the relevant passages | Relevant to claim No. |
|---|---|---|
| PX | CN 113663622 A (VINNO TECHNOLOGY (SUZHOU) CO., LTD.) 19 November 2021 (2021-11-19)<br>claims 1-10 | 1-10 |
| A | CN 105496455 A (VINNO TECHNOLOGY (SUZHOU) CO., LTD.) 20 April 2016 (2016-04-20)<br>abstract, and claim 1 | 1-10 |
| A | CN 103235041 A (XI'AN JIAOTONG UNIVERSITY) 07 August 2013 (2013-08-07)<br>entire document | 1-10 |
| A | CN 108827876 A (INSTITUTE OF ACOUSTICS, CHINESE ACADEMY OF SCIENCES) 16 November 2018 (2018-11-16)<br>entire document | 1-10 |
| A | CN 102249367 A (HOHAI UNIVERSITY, CHANGZHOU) 23 November 2011 (2011-11-23)<br>entire document | 1-10 |
| A | CN 111220700 A (NORTH UNIVERSITY OF CHINA) 02 June 2020 (2020-06-02)<br>entire document | 1-10 |

☑ Further documents are listed in the continuation of Box C. ☑ See patent family annex.

| * | Special categories of cited documents: | "T" | later document published after the international filing date or priority date and not in conflict with the application but cited to understand the principle or theory underlying the invention |
|---|---|---|---|
| "A" | document defining the general state of the art which is not considered to be of particular relevance | | |
| "E" | earlier application or patent but published on or after the international filing date | "X" | document of particular relevance; the claimed invention cannot be considered novel or cannot be considered to involve an inventive step when the document is taken alone |
| "L" | document which may throw doubts on priority claim(s) or which is cited to establish the publication date of another citation or other special reason (as specified) | "Y" | document of particular relevance; the claimed invention cannot be considered to involve an inventive step when the document is combined with one or more other such documents, such combination being obvious to a person skilled in the art |
| "O" | document referring to an oral disclosure, use, exhibition or other means | | |
| "P" | document published prior to the international filing date but later than the priority date claimed | "&" | document member of the same patent family |

| Date of the actual completion of the international search | Date of mailing of the international search report |
|---|---|
| **24 August 2022** | **05 September 2022** |

| Name and mailing address of the ISA/CN | Authorized officer |
|---|---|
| **China National Intellectual Property Administration (ISA/CN)**<br>**No. 6, Xitucheng Road, Jimenqiao, Haidian District, Beijing 100088, China** | |
| Facsimile No. **(86-10)62019451** | Telephone No. |

Form PCT/ISA/210 (second sheet) (January 2015)

**INTERNATIONAL SEARCH REPORT**

| International application No. |
| --- |
| **PCT/CN2022/097141** |

**C.     DOCUMENTS CONSIDERED TO BE RELEVANT**

| Category* | Citation of document, with indication, where appropriate, of the relevant passages | Relevant to claim No. |
| --- | --- | --- |
| A | US 2015141734 A1 (INSTITUT NATIONAL DE LA SANTE ET DE LA RE CHERCHE MEDICALE) 21 May 2015 (2015-05-21)<br>      entire document | 1-10 |

Form PCT/ISA/210 (second sheet) (January 2015)

**INTERNATIONAL SEARCH REPORT**
Information on patent family members

| International application No. |
| --- |
| **PCT/CN2022/097141** |

| Patent document cited in search report | | | Publication date (day/month/year) | Patent family member(s) | | | Publication date (day/month/year) |
| --- | --- | --- | --- | --- | --- | --- | --- |
| CN | 113663622 | A | 19 November 2021 | None | | | |
| CN | 105496455 | A | 20 April 2016 | US | 2017164932 | A1 | 15 June 2017 |
| | | | | US | 10456117 | B2 | 29 October 2019 |
| | | | | CN | 105496455 | B | 28 September 2018 |
| CN | 103235041 | A | 07 August 2013 | CN | 103235041 | B | 02 March 2016 |
| CN | 108827876 | A | 16 November 2018 | CN | 108827876 | B | 28 April 2020 |
| CN | 102249367 | A | 23 November 2011 | CN | 102249367 | B | 24 April 2013 |
| CN | 111220700 | A | 02 June 2020 | None | | | |
| US | 2015141734 | A1 | 21 May 2015 | EP | 2636428 | A1 | 11 September 2013 |
| | | | | WO | 2013132060 | A1 | 12 September 2013 |
| | | | | EP | 2822652 | A1 | 14 January 2015 |
| | | | | ES | 2585049 | T3 | 03 October 2016 |
| | | | | CA | 2865038 | A1 | 12 September 2013 |

Form PCT/ISA/210 (patent family annex) (January 2015)

**EP 4 393 582 A1**

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- CN 202110970195